(19) **Europäisches Patentamt / European Patent Office / Office européen des brevets**

(11) **EP 2 345 891 A1**

(12) **EUROPEAN PATENT APPLICATION**
published in accordance with Art. 153(4) EPC

(43) Date of publication:
**20.07.2011 Bulletin 2011/29**

(21) Application number: **08877144.9**

(22) Date of filing: **01.10.2008**

(51) Int Cl.:
**G01N 25/18** (2006.01)

(86) International application number:
**PCT/JP2008/067811**

(87) International publication number:
**WO 2010/038285 (08.04.2010 Gazette 2010/14)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Yamatake Corporation**
**Tokyo 100-6419 (JP)**

(72) Inventors:
• **OOISHI, Yasuharu**
  **Tokyo 100-6419 (JP)**

• **HAYASHI, Yasue**
  **Tokyo 100-6419 (JP)**
• **MUTO, Hiroyuki**
  **Tokyo 100-6419 (JP)**
• **AOSHIMA, Shigeru**
  **Tokyo 100-6419 (JP)**
• **MORIO, Shuji**
  **Tokyo 100-6419 (JP)**

(74) Representative: **Kling, Simone**
**Lavoix Munich**
**Bayerstrasse 85a**
**80335 München (DE)**

(54) **CALORIFIC VALUE COMPUTATION FORMULA GENERATION SYSTEM, CALORIFIC VALUE COMPUTATION FORMULA GENERATION METHOD, CALORIFIC VALUE COMPUTATION SYSTEM, AND CALORIFIC VALUE COMPUTATION METHOD**

(57)    A calorific value calculation formula generating system is equipped with a measuring mechanism for measuring heat dissipation constant values or thermal conductivity values of each of plural mixed gases each containing plural kinds of gas components at plural temperatures; and a formula generation module for generating a calorific value calculation formula having heat dissipation constant or thermal conductivities at the plural temperatures as independent variables and a calorific value as a dependent variable on the basis of calorific value values of the plural mixed gases and the heat dissipation constant values or the thermal conductivity values measured at the plural temperatures.

FIG.1

**Description**

[Technical Field]

The present invention relates to a gas inspection technique and, more particularly, relates to a calorific value calculation formula generating system, a calorific value calculation formula generating method, a calorific value calculating system, and a calorific value calculating method.

[Background Art]

Conventionally, to determine a calorific value of a mixed gas, it is necessary to analyze components of the mixed gas using an expensive gas chromatography instrument or the like. Another method has been proposed which determines a calorific value of a mixed gas by calculating proportions of components of the mixed gas that are methane ($CH_4$), propane ($C_3H_8$), nitrogen ($N_2$), and carbon dioxide ($CO_2$) by measuring thermal conductivity and a sound speed of the mixed gas. However, this method requires an expensive sound speed sensor for measuring a sound speed as well as a sensor for measuring thermal conductivity. (JP-T-2004-514138 (the symbol "JP-T" as used herein means a published Japanese translation of a PCT application)).

[Disclosure of the Invention]

[Problems to be solved by the invention]

Furthermore, a desire to detect a calorific value of a gas in real time is increasing and apparatus for detecting a calorific value are now required to be higher in processing speed and smaller in size than before. In this connection, a large amount of calculation directly limits the increase in processing speed and the reduction in size of apparatus. However, conventionally, calculation of a calorific value of a mixed gas requires a step of calculating proportions of respective gas components of the mixed gas and a step of calculating a calorific value of the mixed gas on the basis of the calculated proportions, resulting in a problem that the amount of calculation is large. An improved detection method is therefore necessary which can make the amount of calculation lower than in conventional methods. The present invention thus provides a calorific value detecting method and apparatus in which the amount of calculation is smaller than in conventional methods and apparatus.

[Means for solving the problems]

As described above, conventionally, calculation of a calorific value of a mixed gas requires the step of calculating proportions of respective gas components of the mixed gas. In contrast, the inventors reconsidered the calculation method of the calorific value and studied whether a calorific value of a mixed gas can be calculated without the need for executing the step of calculating proportions of its gas components. And the inventors have found theoretically and experimentally a method capable of calculating a calorific value of a mixed gas uniquely using heat dissipation constant or thermal conductivities of the mixed gas as input information.

One aspect of the invention provides a calorific value calculation formula generating system comprising a measuring mechanism for measuring, at plural temperatures, heat dissipation constant values or thermal conductivity values of each of plural mixed gases each containing plural kinds of gas components; and a formula generation module for generating a calorific value calculation formula having heat dissipation constant or thermal conductivities at the plural temperatures as independent variables and a calorific value as a dependent variable on the basis of known calorific value values of the plural mixed gases and the heat dissipation constant values or the thermal conductivity values measured at the plural temperatures.

Another aspect of the invention provides a calorific value calculation formula generating method comprising the steps of preparing plural mixed gases each containing plural kinds of gas components; measuring heat dissipation constant values or thermal conductivity values of each of the plural mixed gases at plural temperatures; and generating a calorific value calculation formula having heat dissipation constant or thermal conductivities at the plural temperatures as independent variables and a calorific value as a dependent variable on the basis of known calorific value values of the plural mixed gases and the heat dissipation constant values or the thermal conductivity values measured at the plural temperatures.

The calorific value calculation formula generating system and the calorific value calculation formula generating method according to the above aspects of the invention provide a calorific value calculation formula which makes it possible to calculate a calorific value of a mixed gas whose calorific value is unknown from heat dissipation constant or thermal conductivities of the mixed gas without the need for executing the step of calculating proportions of its gas components.

Still another aspect of the invention provides a calorific value calculating system comprising a measuring mechanism for measuring, at plural temperatures, heat dissipation constant values or thermal conductivity values of a measurement subject mixed gas whose calorific value is unknown; a formula storage device for storing a calorific value calculation formula having heat dissipation constant or thermal conductivities at the plural temperatures as independent variables and a calorific value as a dependent variable; and a calorific value calculation module for calculating a calorific value of the measurement subject mixed gas by substituting the heat dissipation constant values or the thermal conductivity values of the measurement subject mixed gas measured at the plural temperatures into the heat dissipation constant or the thermal conductivities at the plural temperatures as the independent variables of the calorific value calculation formula.

A further aspect of the invention provides a calorific value calculating method comprising the steps of measuring, at plural temperatures, heat dissipation constant values or thermal conductivity values of a measurement subject mixed gas whose calorific value is unknown; preparing a calorific value calculation formula having heat dissipation constant or thermal conductivities at the plural temperatures as independent variables and a calorific value as a dependent variable; and calculating a calorific value of the measurement subject mixed gas by substituting the heat dissipation constant values or the thermal conductivity values of the measurement subject mixed gas measured at the plural temperatures into the heat dissipation constant or the thermal conductivities at the plural temperatures as the independent variables of the calorific value calculation formula.

The calorific value calculating system and the calorific value calculating method according to the above aspects of the invention make it possible to calculate a calorific value of a mixed gas whose calorific value is unknown by measuring heat dissipation constant or thermal conductivities of the mixed gas without the need for executing the step of calculating proportions of its gas components.

[Advantages of the invention]

The invention can provide a calorific value calculation formula generating system, a calorific value calculation formula generating method, a calorific value calculating system, and a calorific value calculating method which make it possible to calculate a calorific value with a small amount of calculation.

[Brief Description of the Drawings]

[Fig. 1] Fig. 1 is a perspective view of a microheater according to a first embodiment of the present invention.

[Fig. 2] Fig. 2 is a sectional view, taken along line II-II in Fig. 1, of the microheater according to the first embodiment of the invention.

[Fig. 3] Fig. 3 is a graph showing relationships between the heating temperature of a heating resistor according to the first embodiment of the invention and the heat dissipation constant of respective gases.

[Fig. 4] Fig. 4 is a first schematic diagram of a calorific value calculation formula generating system according to the first embodiment of the invention.

[Fig. 5] Fig. 5 is a second schematic diagram of the calorific value calculation formula generating system according to the first embodiment of the invention.

[Fig. 6] Fig. 6 is a flowchart showing a calorific value calculation formula generating method according to the first embodiment of the invention.

[Fig. 7] Fig. 7 is a schematic diagram of a calorific value calculating system according to a second embodiment of the invention.

[Fig. 8] Fig. 8 is a flowchart showing a calorific value calculating method according to the second embodiment of the invention.

[Fig. 9] Fig. 9 is a table showing compositions and calorific values of sample mixed gases used in Example of the embodiments of the invention.

[Fig. 10] Fig. 10 is a graph showing calculated calorific values and the true calorific values of the sample mixed gases used in Example of the embodiments of the invention.

[Fig. 11] Fig. 11 is a graph showing a relationship between the calculated calorific values and the true calorific values of the sample mixed gases used in Example of the embodiments of the invention.

[Fig. 12] Fig. 12 is a graph showing a relationship between the thermal conductivity and the heat dissipation constant according to another embodiment of the invention.

[Best Mode for Carrying Out the Invention]

Embodiments of the present invention will be hereinafter described. In the following drawings, the same or similar things are denoted by the same symbol or similar symbols. The drawings are schematic and hence specific dimensions etc. should be recognized in conjunction with the following description. It goes without saying that different drawings may include members that are drawn in such a manner that relationships or ratios between their dimensions are different from each other.

(First embodiment)

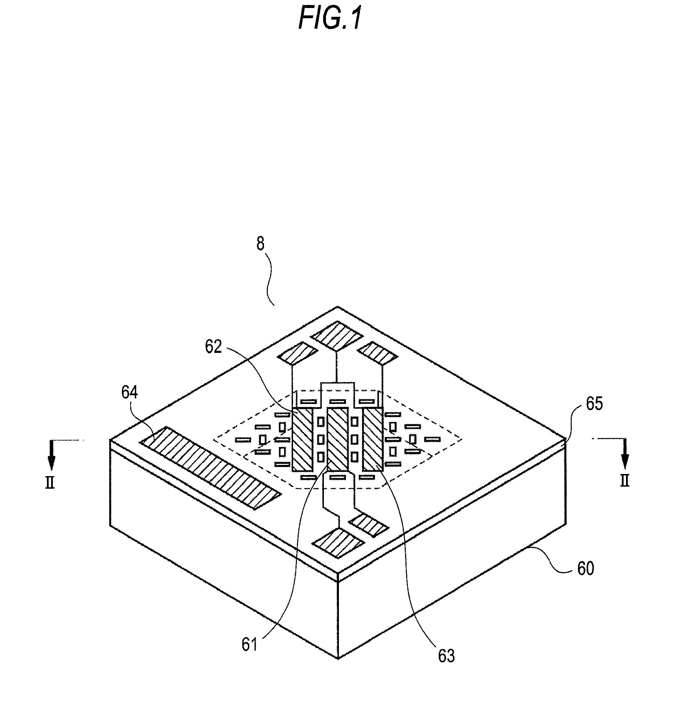

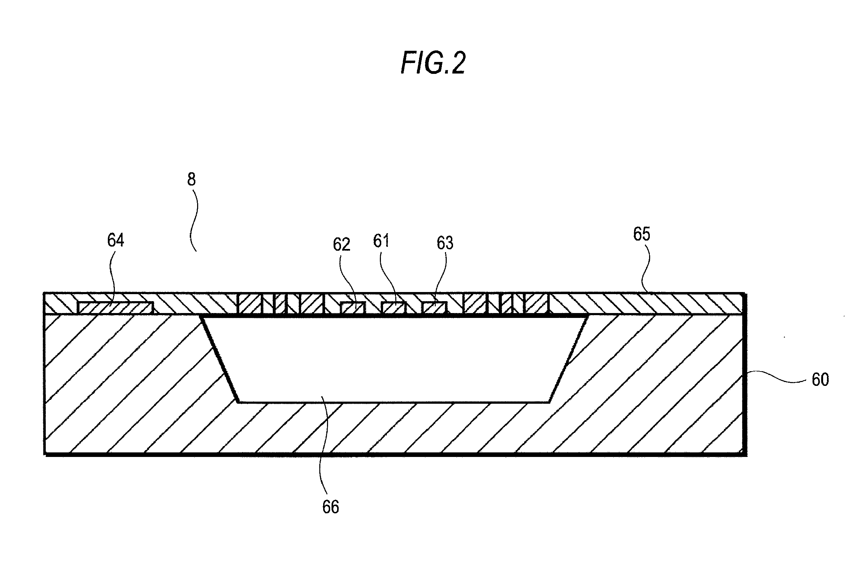

First, a microheater 8 which is used in a calorific value calculation formula generating system and a calorific value calculation formula generating method according to a first embodiment will be described with reference to a perspective view of Fig. 1 and a sectional view of Fig. 2 taken along line II-II. The microheater 8 is equipped with a substrate 60 having a cavity 66 and an insulating film 65 which is disposed so as to cover the cavity 66 on the substrate 60. The thickness of the substrate 60 is 0.5 mm, for example. The horizontal and vertical dimensions of the substrate 60 are each about 1.5 mm, for example. That portion of the insulating film 65 which covers the cavity 66 serves as a heat-insulative diaphragm.

The microheater 8 is also equipped with a heating resistor 61 which is provided in the insulating film 65, a first temperature-measuring resistor 62 and a second temperature-measuring resistor 63 which are provided in the insulating film 65 on both sides of the heating resistor 61, and a gas temperature sensor 64 which is provided in the insulating film 65 so as to be spaced from the heating resistor 61. Disposed at the center of the insulating film 65 which covers the cavity 66, the heating resistor 61 heats an atmosphere gas adjacent to it. Provided in the insulating film 65 so as to be spaced from the heating resistor 61, the gas temperature sensor 64 detects a temperature of the atmosphere gas without being influenced by the temperature of the heating resistor 61.

Silicon (Si) etc. can be used as a material of the substrate 60. Silicon dioxide ($SiO_2$) can be used as a material of the insulating film 65. The cavity 66 is formed by anisotropic etching or the like. Platinum (Pt) etc. can be used as materials of the heating resistor 61, the first temperature-measuring resistor 62, the second temperature-measuring resistor 63, and the gas temperature sensor 64, and they can be formed by lithography or the like.

The resistance of the heating resistor 61 varies with the temperature. The relationship between the temperature $T_H$ and the resistance $R_H$ of the heating resistor 61 is given by the following Equation (1):

$$R_H = R_{STD} \times \{1 + \alpha(T_H - T_{STD}) + \beta(T_H - T_{STD})^2\} \quad \cdots (1)$$

where $T_{STD}$ is a standard temperature (e.g., 20°C), $R_{STD}$ is a resistance value measured in advance at the standard temperature $T_{STD}$, $\alpha$ is a first-order temperature coefficient of the resistance, and $\beta$ is a second-order temperature coefficient of the resistance. The resistance $R_H$ of the heating resistor 61 is also given by the following Equation (2) as a function of the drive power $P_H$ and the conduction current $I_H$ of the heating resistor 61:

$$R_H = P_H/I_H{}^2 \quad \cdots (2)$$

Furthermore, the resistance $R_H$ of the heating resistor 61 is given by the following Equation (3) as a function of the voltage $V_H$ across the heating resistor 61 and the conduction current $I_H$ flowing through the heating resistor 61:

$$R_H = V_H/I_H \quad \cdots (3)$$

The temperature $T_H$ of the heating resistor 61 is stabilized when thermal equilibrium is established between the heating resistor 61 and the atmosphere gas. In an equilibrium state, the heat dissipation constant $M_O$ of the atmosphere gas is obtained by dividing the drive power $P_H$ of the heating resistor 61 by the difference between the temperature $T_H$ of the heating resistor 61 and the temperature $T_O$ of the atmosphere gas as indicated by the following Equation (4). The unit of the heat dissipation constant $M_O$ is W/°C, for example.

$$M_O = P_H/(T_H - T_O) \quad \cdots (4)$$

Since the conduction current $I_H$, the drive power $P_H$, and the voltage $V_H$ of the heating resistor 61 can be measured, the temperature $T_H$ of the heating resistor 61 can be calculated according to the above Equations (1)-(3). The Temperature $T_O$ of the atmosphere gas can be measured by the gas temperature sensor 64 shown in Fig. 1. Therefore, the heat dissipation constant $M_O$ of the atmosphere gas can be calculated by using the microheater 8 shown in Figs. 1 and 2.

Then, it is assumed that the atmosphere gas is a mixed gas of four gas components, that is, gas A, gas B, gas C, and gas D. The sum of volume proportions $V_A$, $V_B$, $V_C$, and $V_D$ of gas A, gas B, gas C, and gas D, respectively, is equal to 1 as indicated by the following Equation (5):

$$V_A + V_B + V_C + V_D = 1 \qquad \cdots (5)$$

Let $K_A$, $K_B$, $K_C$, and $K_D$ represent the calorific values per unit volume of gas A, gas B, gas C, and gas D, respectively; then, the calorific value Q per unit volume of the mixed gas is equal to the sum of the products of the calorific values per unit volume of the respective gas components and the volume proportions of the respective gas components. That is, the calorific value Q per unit volume of the mixed gas is given by the following Equation (6). The unit of the calorific value per unit volume is MJ/m$^3$, for example.

$$Q = K_A \times V_A + K_B \times V_B + K_C \times V_C + \times K_D \times V_D \qquad \cdots (6)$$

Let $M_A$, $M_B$, $M_C$, and $M_D$ represent the heat dissipation constant of gas A, gas B, gas C, and gas D, respectively; then, the heat dissipation constant $M_I$ of the mixed gas is equal to the sum of the products of the heat dissipation constant of the respective gas components and the volume proportions of the respective gas components. That is, the heat dissipation constant $M_I$ of the mixed gas is given by the following Equation (7):

$$M_I = M_A \times V_A + M_B \times V_B + M_C \times V_C + \times M_D \times V_D \qquad \cdots (7)$$

Since the heat dissipation constant of a gas depends on the temperature T of the heating resistor 61, the heat dissipation constant $M_I$ of the mixed gas is given by the following Equation (8) which is a function of the temperature T of the heating resistor 61:

$$M_I(T) = M_A(T) \times V_A + M_B(T) \times V_B + M_C(T) \times V_C$$
$$+ \times M_D(T) \times V_D \qquad \cdots (8)$$

Therefore, the heat dissipation constant $M_I(T_1)$ of the mixed gas when the temperature of the heating resistor 61 is $T_1$ is given by the following Equation (9), the heat dissipation constant $M_I(T_2)$ of the mixed gas when the temperature of the heating resistor 61 is $T_2$ is given by the following Equation (10), and the heat dissipation constant $M_I(T_3)$ of the mixed gas when the temperature of the heating resistor 61 is $T_3$ is given by the following Equation (11). The temperatures $T_1$, $T_2$, and $T_3$ are different temperatures.

$$M_I(T_1) = M_A(T_1) \times V_A + M_B(T_1) \times V_B + M_C(T_1) \times V_C$$
$$+ \times M_D(T_1) \times V_D \qquad \cdots (9)$$

$$M_I(T_2) = M_A(T_2) \times V_A + M_B(T_2) \times V_B + M_C(T_2) \times V_C$$
$$+ \times M_D(T_2) \times V_D \qquad \cdots (10)$$

$$M_I(T_3) = M_A(T_3) \times V_A + M_B(T_3) \times V_B + M_C(T_3) \times V_C$$

$$+ \times M_D(T_3) \times V_D \qquad \cdots (11)$$

Where the heat dissipation constant $M_A(T)$, $M_B(T)$, $M_C(T)$, and $M_D(T)$ of the respective gas components are nonlinear with respect to the temperature T of the heating resistor 61, the above Equations (9)-(11) are linearly independent of each other. Even where the heat dissipation constant $M_A(T)$, $M_B(T)$, $M_C(T)$, and $M_D(T)$ of the respective gas components are linear with respect to the temperature T of the heating resistor 61, the Equations (9)-(11) are linearly independent of each other if the heat dissipation constant $M_A(T)$, $M_B(T)$, $M_C(T)$, and $M_D(T)$ of the respective gas components have different variation rates with respect to the temperature T of the heating resistor 61. Furthermore, where Equations (9)-(11) are linearly independent of each other, Equations (5) and (9)-(11) are also linearly independent of each other.

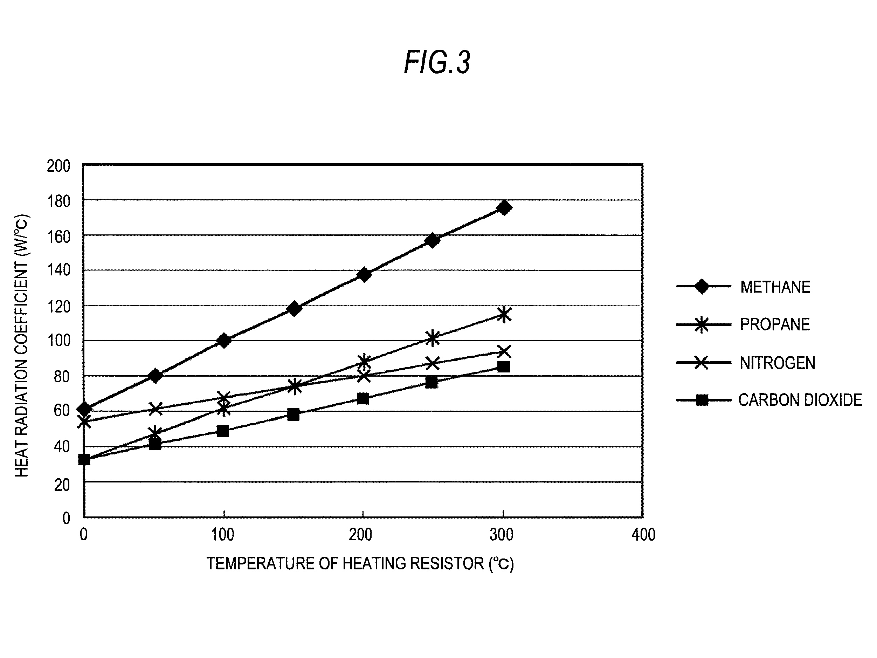

Fig. 3 is a graph showing a relationship between the temperature of the heating resistor 61 and the heat dissipation constant of methane ($CH_4$), propane ($C_3H_8$), nitrogen ($N_2$), and carbon dioxide ($CO_2$) which are included in a natural gas. The heat dissipation constant of the gas components methane ($CH_4$), propane ($C_3H_8$), nitrogen ($N_2$), and carbon dioxide ($CO_2$) are linear with respect to the temperature of the heating resistor 61. However, the heat dissipation constant of methane ($CH_4$), propane ($C_3H_8$), nitrogen ($N_2$), and carbon dioxide ($CO_2$) have different variation rates with respect to the temperature of the heating resistor 61. Therefore, where the gas components constituting a mixed gas are methane ($CH_4$), propane ($C_3H_8$), nitrogen ($N_2$), and carbon dioxide ($CO_2$), Equations (9)-(11) are linearly independent of each other.

The values of the heat dissipation constant $M_A(T_1)$, $M_B(T_1)$, $M_C(T1)$, $M_D(T_1)$, $M_A(T_2)$, $M_B(T_2)$, $M_C(T_2)$, $M_D(T_2)$, $M_A(T_3)$, $M_B(T_3)$, $M_C(T_3)$, and $M_D(T_3)$ of the gas components in Equations (9)-(11) can be acquired in advance by measurements or the like. Therefore, the volume proportions $V_A$, $V_B$, $V_c$, and $V_D$ of gas A, gas B, gas C, and gas D can be obtained as the following Equations (12)-(15), respectively, which are functions of the heat dissipation constant $M_I(T_1)$, $M_I(T_2)$, and $M_I(T_3)$ of the mixed gas by solving the simultaneous equations of Equations (5) and (9)-(11). In the following Equations (12)-(15), $f_n$ is a symbol representing a function and n is a natural number.

$$V_A = f_1\{M_I(T_1),\, M_I(T_2),\, M_I(T_3)\} \qquad \cdots (12)$$

$$V_B = f_2\{M_I(T_1),\, M_I(T_2),\, M_I(T_3)\} \qquad \cdots (13)$$

$$V_C = f_3\{M_I(T_1),\, M_I(T_2),\, M_I(T_3)\} \qquad \cdots (14)$$

$$V_D = f_4\{M_I(T_1),\, M_I(T_2),\, M_I(T_3)\} \qquad \cdots (15)$$

The following Equation (16) is obtained by substituting Equations (12)-(15) into the above-mentioned Equation (6):

$$Q = K_A \times V_A + K_B \times V_B + K_C \times V_C + \times K_D \times V_D$$

$$= K_A \times f_1\{M_I(T_1),\, M_I(T_2),\, M_I(T_3)\} + K_B \times f_2\{M_I(T_1),\, M_I(T_2),\, M_I(T_3)\} + K_C \times$$

$$f_3\{M_I(T_1),\, M_I(T_2),\, M_I(T_3)\} + K_D \times f_4\{M_I(T_1),\, M_I(T_2),\, M_I(T_3)\} \qquad \cdots (16)$$

As seen from the above Equation (16), the calorific value Q per unit volume of the mixed gas is given by the equation having, as variables, the heat dissipation constant $M_I(T_1)$, $M_I(T_2)$, and $M_I(T_3)$ of the mixed gas at the temperatures $T_1$, $T_2$, and $T_3$. Therefore, the calorific value Q of the mixed gas is given by the following Equation (17) in which symbol g represents a function:

$$Q = g\{M_I(T_1), M_I(T_2), M_I(T_3)\} \quad \cdots (17)$$

The inventors have thus found that if Equation (17) is obtained in advance for mixed gas consisting of gas A, gas B, gas C, and gas D, a calorific value Q per unit volume of a test subject mixed gas whose volume proportions of $V_A$ of gas A, $V_B$ of gas B, $V_C$ of gas C, and $V_D$ of gas D, are unknown can be determined uniquely by measuring heat dissipation constant $M_I(T_1)$, $M_I(T_2)$, and $M_I(T_3)$ of the test subject mixed gas at the temperatures $T_1$, $T_2$, and $T_3$ and substituting them into Equation (17).

The number of kinds of gas components of a mixed gas is not limited to four. If the following Equation (18) is obtained in advance as an equation having, as variables, the heat dissipation constant $M_I(T_1)$, $M_I(T_2)$, $M_I(T_3)$, $\cdots$, $M_I(T_{n-1})$ at at least n-1 temperatures $T_1$, $T_2$, $T_3$, $\cdots$, $T_{n-1}$ of mixed gas consisting of n kinds of component gases, a calorific value Q per unit volume of a test subject mixed gas whose volume proportions of the n kinds of gas components are unknown can be calculated uniquely by measuring heat dissipation constant $M_I(T_1)$, $M_I(T_2)$, $M_I(T_3)$, ..., $M_I(T_{n-1})$ of the test subject mixed gas at the temperatures $T_1$, $T_2$, and $T_3$ and substituting them into Equation (18):

$$Q = g\{M_I(T_1), M_I(T_2), M_I(T_3), \cdots, M_I(T_{n-1})\} \quad \cdots (18)$$

Where the gas components of a mixed gas include, in addition to methane ($CH_4$) and propane ($C_3H_8$), alkanes ($C_jH_{2j+2}$; j is a natural number) other than methane ($CH_4$) and propane ($C_3H_8$), regarding each of the alkanes ($C_jH_{2j+2}$) other than methane ($CH_4$) and propane ($C_3H_8$) as a mixture of methane ($CH_4$) and propane ($C_3H_8$) does not influence the calculation of Equation (18). For example, Equation (18) may be calculated by regarding each of ethane ($C_2H_6$), butane ($C_4H_{10}$), pentane ($C_5H_{12}$), and hexane ($C_6H_{14}$) as a mixture of methane ($CH_4$) and propane ($C_3H_8$) that are multiplied by prescribed coefficients as in the following Equations (19)-(22):

$$C_2H_6 = 0.5CH_4 + 0.5C_3H_8 \quad \cdots (19)$$

$$C_4H_{10} = -0.5CH_4 + 1.5C_3H_8 \quad \cdots (20)$$

$$C_5H_{12} = -1.0CH_4 + 2.0C_3H_8 \quad \cdots (21)$$

$$C_6H_{14} = -1.5CH_4 + 2.5C_3H_8 \quad \cdots (22)$$

Therefore, where a mixed gas consisting of n kinds of gas components contains, in addition to methane ($CH_4$) and propane ($C_3H_8$) as gas components, z kinds of alkanes ($C_jH_{2j+2}$) (z: natural number) other than methane ($CH_4$) and propane ($C_3H_8$), an equation having, as variables, the heat dissipation constant of the mixed gas at least n-z-1 temperatures may be determined.

Naturally, Equation (18) can be used for calculating a calorific value Q of a test subject mixed gas in the case where the kinds of gas components of the test subject mixed gas whose calorific value Q per unit volume is unknown are the same as those of mixed gases that were used for determining Equation (18). Equation (18) can also be used in the case where a test subject mixed gas consists of smaller-than-n kinds of gas components that are contained in mixed gases that were used for determining Equation (18). For example, where each of mixed gases that were used for determining Equation (18) contains four gas components methane ($CH_4$), propane ($C_3H_8$), nitrogen, ($N_2$), and carbon dioxide ($CO_2$), Equation (18) can also be used for calculating a calorific value Q of a test subject mixed gas which contains only three kinds of gas components methane ($CH_4$), propane ($C_3H_8$), and carbon dioxide ($CO_2$) which do not include nitrogen ($N_2$).

Where each of mixed gases that were used for determining Equation (18) contains gas components methane ($CH_4$) and propane ($C_3H_8$) as gas components, Equation (18) can be used even if a test subject mixed gas contains alkanes ($C_jH_{2j+2}$) that are not contained in mixed gases that were used for determining Equation (18). This is because, as described above, regarding each of the alkanes ($C_jH_{2j+2}$) other than methane ($CH_4$) and propane ($C_3H_8$) as a mixture of methane ($CH_4$) and propane ($C_3H_8$) does not influence the calculation of a calorific value Q per unit volume using Equation (18).

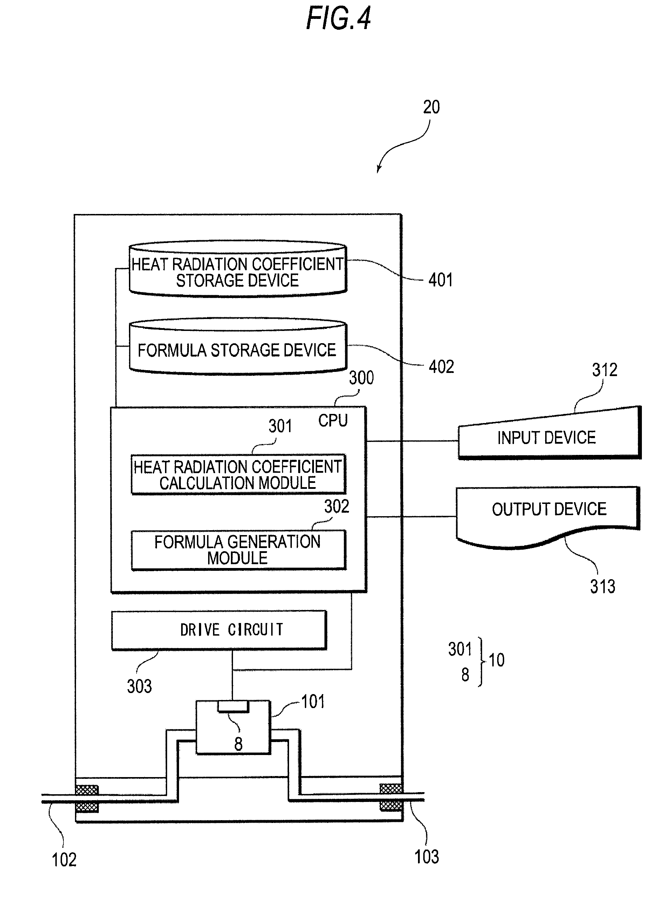

A calorific value calculation formula generating system 20 according to the first embodiment shown in Fig. 4 is equipped with a measuring mechanism 10 for measuring, at plural temperatures, heat dissipation constant values of plural sample mixed gases each of which contains plural kinds of gas components and whose calorific value values are known and a formula generation module 302 for generating a calorific value calculation formula having the heat dissipation constant at plural temperatures as independent variables and the calorific value as a dependent variable on the basis of the known calorific values of the plural sample mixed gases and the heat dissipation constant values measured at the plural temperatures.

The measuring mechanism 10 is equipped with the microheater 8 (described above with reference to Figs. 1 and 2) for heating each of the plural sample mixed gases. The microheater 8 is disposed in a chamber 101 (see Fig. 4) into which each of the plural sample mixed gases are to be injected. Connected to the chamber 101 are a flow passage 102 for sending each of the plural sample mixed gases to the chamber 101 and a flow passage 103 for venting each of the plural sample mixed gases from the chamber 101 to the outside.

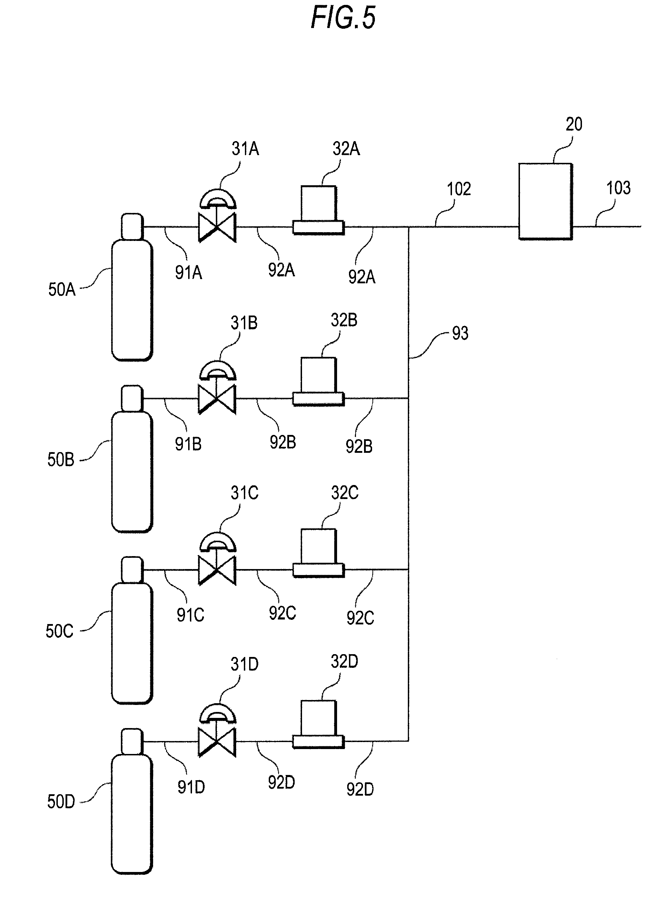

Where four kinds of sample mixed gases are used, a first gas cylinder 50A for storing a first sample mixed gas, a second gas cylinder 50B for storing a second sample mixed gas, a third gas cylinder 50C for storing a third sample mixed gas, and a fourth gas cylinder 50D for storing a fourth sample mixed gas are prepared as shown in Fig. 5. A first gas pressure regulator 31A for obtaining a first sample mixed gas whose pressure is adjusted to as low a value as 0.2 MPa, for example, from the first gas cylinder 50A is connected to the first gas cylinder 50A via a flow passage 91A. A first flow rate controller 32A is connected to the first gas pressure regulator 31A via a flow passage 92A. The first flow rate controller 32A controls the flow rate of the first sample mixed gas being sent to the calorific value calculation formula generating system 20 via the flow passages 92A and 102.

A second gas pressure regulator 31B is connected to the second gas cylinder 50B via a flow passage 91B. A second flow rate controller 32B is connected to the second gas pressure regulator 31B via a flow passage 92B. The second flow rate controller 32B controls the flow rate of the second sample mixed gas being sent to the calorific value calculation formula generating system 20 via the flow passages 92B, 93 and 102.

A third gas pressure regulator 31C is connected to the third gas cylinder 50C via a flow passage 91C. A third flow rate controller 32C is connected to the third gas pressure regulator 31C via a flow passage 92C. The third flow rate controller 32C controls the flow rate of the third sample mixed gas being sent to the calorific value calculation formula generating system 20 via the flow passages 92C, 93 and 102.

A fourth gas pressure regulator 31D is connected to the fourth gas cylinder 50D via a flow passage 91D. A fourth flow rate controller 32D is connected to the fourth gas pressure regulator 31D via a flow passage 92D. The fourth flow rate controller 32D controls the flow rate of the fourth sample mixed gas being sent to the calorific value calculation formula generating system 20 via the flow passages 92D, 93 and 102.

Each of the first to fourth sample mixed gases is a natural gas, for example. Each of the first to fourth sample mixed gases contains four kinds of gas components methane ($CH_4$), propane ($C_3H_8$), nitrogen ($N_2$), and carbon dioxide ($CO_2$), for example.

The heating resistor 61 (see Figs. 1 and 2) of the microheater 8 shown in Fig. 4 is supplied with drive power $P_H$ from a drive circuit 303 shown in Fig. 4. Supplied with drive power $P_H$, the heating resistor 61 (see Figs. 1 and 2) heats at 100°C, 150°C, and 200°C, for example. The gas temperature sensor 64 detects a temperature $T_O$ of each of the first to fourth sample mixed gases.

Where a sample mixed gas contains n kinds of gas components, the heating resistor 61 is caused to heat at at least n-1 temperatures. However, as described above, an alkane ($C_jH_{2j+2}$) other than methane ($CH_4$) and propane ($C_3H_8$) can be regarded as a mixture of methane ($CH_4$) and propane ($C_3H_8$). Therefore, where a sample mixed gas consisting of n kinds of gas components contains, in addition to methane ($CH_4$) and propane ($C_3H_8$) as gas components, z kinds of alkanes ($C_jH_{2j+2}$) (z: natural number), the heating resistor 61 is caused to heat at at least n-z-1 temperatures.

The measuring mechanism 10 shown in Fig. 4 is also equipped with a heat dissipation constant calculation module 301 which is connected to the microheater 8. The heat dissipation constant calculation module 301 calculates heat dissipation constant values of each of the first to fourth sample mixed gases according to the above-mentioned Equation (4) by dividing the drive power $P_H$ of the heating resistor 61 (see Figs. 1 and 2) by the differences between the temperatures $T_H$ of the heating resistor 61 and the temperatures $T_O$ of each of the first to fourth sample mixed gases. For example, where the heating resistor 61 is caused to heat at 100°C, 150°C, and 200°C, a heat dissipation constant value at the heating temperature 100°C, a heat dissipation constant value at the heating temperature 150°C, and a heat dissipation constant value at the heating temperature 200°C are calculated for each of the first to fourth sample mixed gases.

The formula generation module 302 shown in Fig. 4 collects a known calorific value, the heat dissipation constant value at the heating temperature 100°C, the heat dissipation constant value at the heating temperature 150°C, and the heat dissipation constant value at the heating temperature 200°C of each of the first to fourth sample mixed gases, for example. Furthermore, on the basis of the collected calorific value values and heat dissipation constant values, the formula generation module 302 determines a calorific value calculation formula having the heat dissipation constant at the heating temperatures 100°C, 150°C, and 200°C as independent variables and the calorific value as a dependent variable by multivariate analyses including support vector regression disclosed in A.J. Smola and B. Scholkopf, "A Tutorial on Support Vector Regression," NeuroCOLT Technical Report NC-TR-98-030, 1998, multiple repression analysis, and a fuzzy quantification theory type II which is disclosed in JP-A-5-141999. The heat dissipation constant calculation module 301 and the formula generation module 302 are incorporated in a central processing unit (CPU) 300.

The calorific value calculation formula generating system 20 is also equipped with a heat dissipation constant storage device 401 and a formula storage device 402 which are connected to the CPU 300. The heat dissipation constant storage device 401 stores heat dissipation constant values that are calculated by the heat dissipation constant calculation module 301. The formula storage device 402 stores a calorific value calculation formula that is generated by the formula generation module 302. An input device 312 and an output device 313 are also connected to the CPU 300. For example, a keyboard, a pointing device such as a mouse, etc. can be used as the input device 312. An image display device such as a liquid crystal display or a monitor and a printer, etc. can be used as the output device 313.

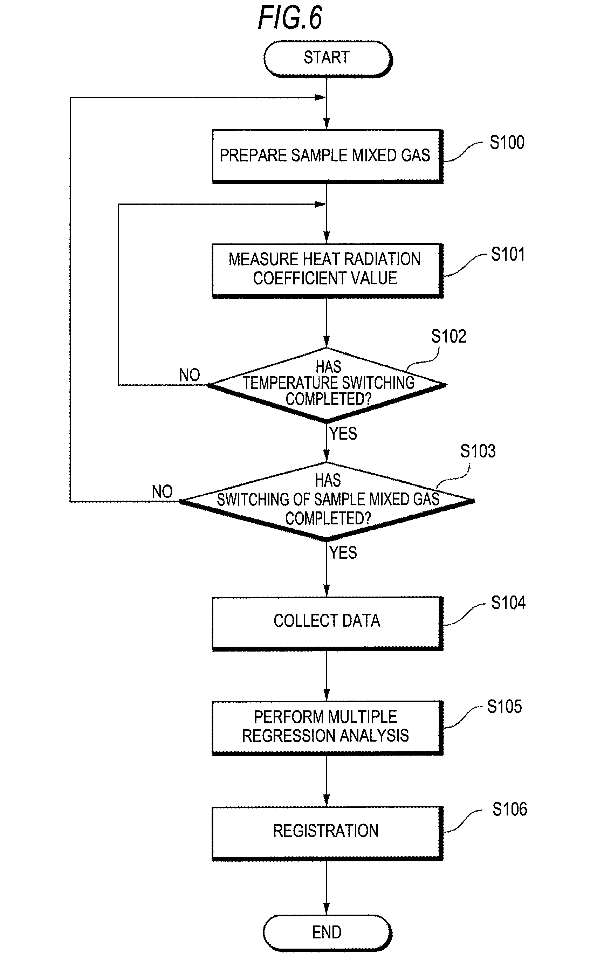

Next, a calorific value calculation formula generating method according to the first embodiment will be described with reference to a flowchart of Fig. 6. In the following example, a description will be made of a case that first to fourth sample mixed gases are prepared and the heating resistor 61 is caused to heat at three temperatures 100°C, 150°C, and 200°C (switching is made).

(a) At step S 100, the first sample mixed gas is inleted into the chamber 101 (see Fig. 4) by opening the valve of the first flow rate controller 32A with the valves of the second to fourth flow rate controllers 32B- 32D kept closed (see Fig. 5). Then, at step S101, the drive circuit 303 causes the heating resistor 61 (see Figs. 1 and 2) to heat at 100°C and the heat dissipation constant calculation module 301 (see Fig. 4) calculates a heat dissipation constant value at the heating temperature 100°C. Then, the heat dissipation constant calculation module 301 stores, in the heat dissipation constant storage device 401, the heat dissipation constant value of the first sample mixed gas at the heating temperature 100°C.

(b) At step S102, it is judged whether the switching of the heating temperature of the heating resistor 61 (see Figs. 1 and 2) has completed. Since switching to the heating temperature 150°C or the heating temperature 200°C has not been made yet, the process returns to step S101, where the drive circuit 303 causes the heating resistor 61 (see Figs. 1 and 2) to heat at 150°C. The heat dissipation constant calculation module 301 (see Fig. 4) calculates a heat dissipation constant value of the first sample mixed gas at the heating temperature 150°C and stores it in the heat dissipation constant storage device 401.

(c) Again at step S102, it is judged whether the switching of the heating temperature of the heating resistor 61 (see Figs. 1 and 2) has completed. Since switching to the heating temperature 200°C has not been made yet, the process returns to step S101, where the drive circuit 303 causes the heating resistor 61 (see Figs. 1 and 2) to heat at 200°C. The heat dissipation constant calculation module 301 (see Fig. 4) calculates a heat dissipation constant value of the first sample mixed gas at the heating temperature 200°C and stores it in the heat dissipation constant storage device 401.

(d) Since the switching of the heating temperature of the heating resistor 61 (see Figs. 1 and 2) has completed, the process moves from step S102 to step S103, where it is judged whether the switching of the sample mixed gas has completed. Since switching to the second, third, or fourth sample mixed gas has not been made yet, the process returns to step S100. At step S100, the second sample mixed gas is inleted into the chamber 101 (see Fig. 4) by closing the first flow rate controller 32A and opening the valve of the second flow rate controller 32B with the valves of the third and fourth flow rate controllers 32C and 32D kept closed (see Fig. 5).

(e) As in the case of the first sample mixed gas, as the loop of steps S101 and S102 is executed repeatedly, the heat dissipation constant calculation module 301 (see Fig. 4) calculates a heat dissipation constant value of the second sample mixed gas at the heating temperature 100°C, a heat dissipation constant value of the second sample mixed gas at the heating temperature 150°C, and a heat dissipation constant value of the second sample mixed gas at the heating temperature 200°C and stores them in the heat dissipation constant storage device 401.

(f) Then, as the loop of steps S100-S103 is executed repeatedly, heat dissipation constant values of the third sample mixed gas at the heating temperatures 100°C, 150°C, and 200°C and heat dissipation constant values of the fourth sample mixed gas at the heating temperatures 100°C, 150°C, and 200°C are stored in the heat dissipation constant storage device 401.

(g) At step S104, a known calorific value of the first sample mixed gas, a known calorific value of the second sample mixed gas, a known calorific value of the third sample mixed gas, and a known calorific value of the fourth sample mixed gas are input from the input device 312 to the formula generation module 302. The formula generation module 302 reads out the heat dissipation constant values of the first to fourth sample mixed gases at each of the heating temperatures 100°C, 150°C, and 200°C from the heat dissipation constant storage device 401.

(h) At step S105, on the basis of the calorific values of the first to fourth sample mixed gases and the heat dissipation constant values of the first to fourth sample mixed gases at each of the heating temperatures 100°C, 150°C, and 200°C, the formula generation module 302 determines a calorific value calculation formula having the heat dissipation constant at the heating temperature 100°C, the heat dissipation constant at the heating temperature 150°C, and the heat dissipation constant at the heating temperature 200°C as independent variables and the calorific value as a dependent variable. Then, at step S106, the formula generation module 302 stores the generated calorific value calculation formula in the formula storage device 402. The calorific value calculation formula generating method according to the first embodiment is thus finished.

As described above, the calorific value calculation formula generating system and method according to the first embodiment make it possible to generate a calorific value calculation formula according to which a calorific value of a measurement subject mixed gas can be calculated uniquely by measuring, at plural temperatures, heat dissipation constant of the measurement subject mixed gas whose calorific value is unknown.

(Second embodiment)

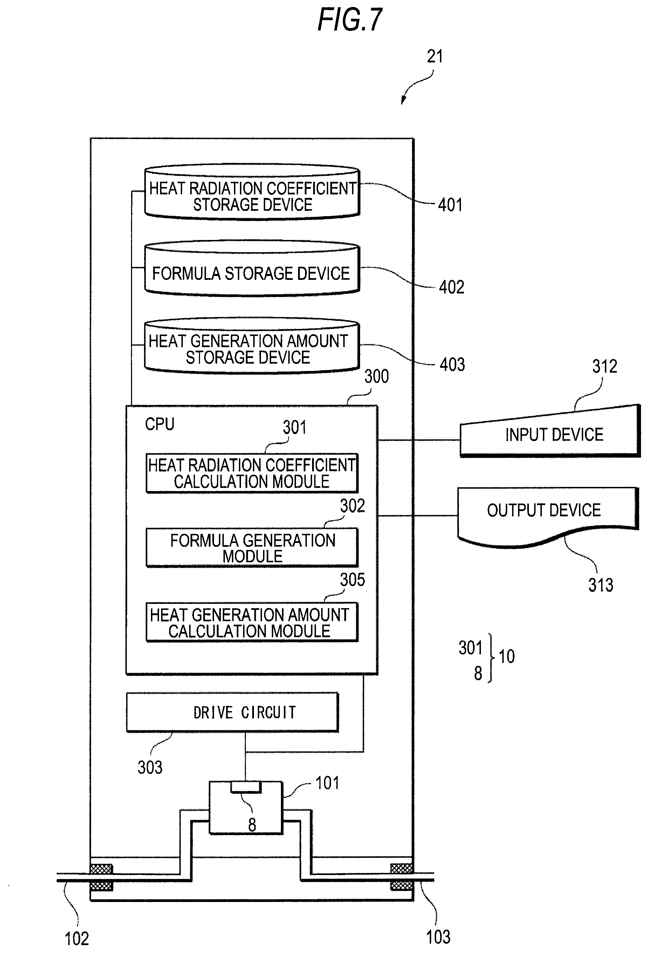

As shown in Fig. 7, a calorific value calculating system 21 according to a second embodiment is equipped with a measuring mechanism 10 for measuring, at plural temperatures, heat dissipation constant values of a measurement subject mixed gas whose calorific value is unknown, a formula storage device 402 for storing a calorific value calculation formula having the heat dissipation constant at the plural temperatures as independent variables and the calorific value as a dependent variable, and a calorific value calculation module 305 for calculating a calorific value of the measurement subject mixed gas by substituting the heat dissipation constant values of the measurement subject mixed gas measured at the plural temperatures into the heat dissipation constant (independent variables) at the plural temperatures of the calorific value calculation formula.

As described in the first embodiment, the formula storage device 402 is stored with a calorific value calculation formula that was generated in the manner described in the first embodiment. In this embodiment, a description will be made of an example in which natural gases each containing methane ($CH_4$), propane ($C_3H_8$), nitrogen ($N_2$), and carbon dioxide ($CO_2$) were used as sample mixed gases to generate the calorific value calculation formula. It is assumed that the calorific value calculation formula has, as independent variables, the heat dissipation constant at a heating temperature 100°C, the heat dissipation constant at a heating temperature 150°C, and the heat dissipation constant at a heating temperature 200°C.

In the second embodiment, for example, a natural gas containing methane ($CH_4$), propane ($C_3H_8$), nitrogen ($N_2$), and carbon dioxide ($CO_2$) at unknown volume proportions is inleted into the chamber 101 as a measurement subject mixed gas. The heating resistor 61 (see Figs. 1 and 2) in the chamber 101 heats at the same three heating temperatures 100°C, 150°C, and 200°C as used in generating the calorific value calculation formula and heats the measurement subject mixed gas.

The heat dissipation constant calculation module 301 shown in Fig. 7 calculates heat dissipation constant values of the measurement subject mixed gas at the heating temperatures 100°C, 150°C, and 200°C according to the method that was explained above by using Equations (1)-(4). The calorific value calculation module 305 calculates a calorific value of the measurement subject mixed gas by substituting the calculated heat dissipation constant values of the measurement subject mixed gas at the heating temperatures 100°C, 150°C, and 200°C into the heat dissipation constant (independent variables) of the calorific value calculation formula.

A calorific value storage device 403 is also connected to the CPU 300. The calorific value storage device 403 stores the calorific value of the measurement subject mixed gas that has been calculated by the calorific value calculation module 305. The other constituent elements of the calorific value calculating system according to the second embodiment are the same as the corresponding ones of the calorific value calculation formula generating system according to the first embodiment described above with reference to Fig. 4 and hence will not be described.

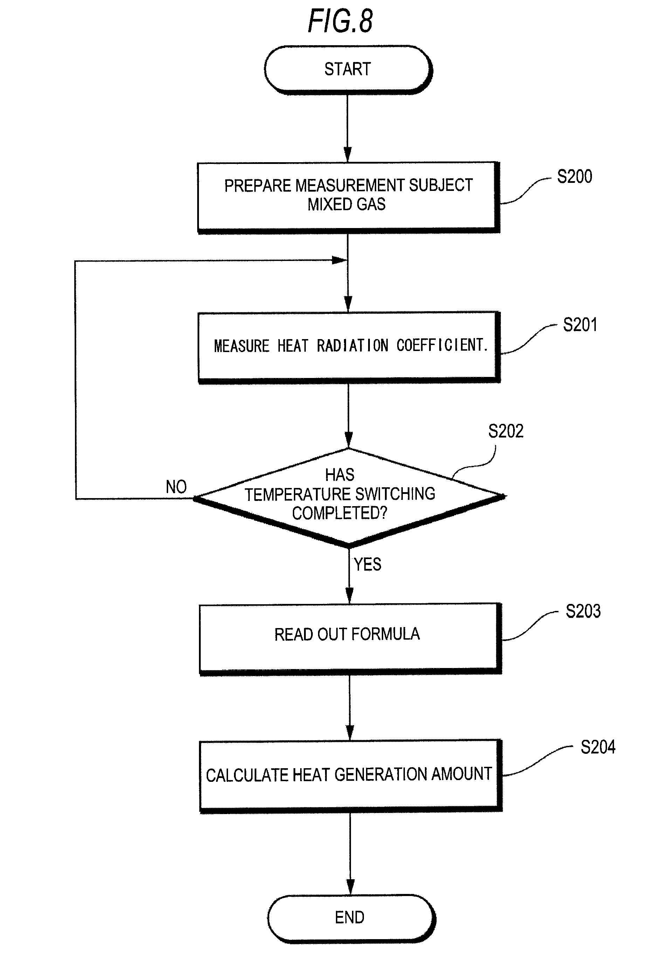

Next, a calorific value calculating method according to the second embodiment will be described with reference to a flowchart of Fig. 8. The following example is such that the heating resistor 61 is caused to heat at three temperatures 100°C, 150°C, and 200°C (switching is made).

(a) At step S200, a measurement subject mixed gas is inleted into the chamber 101 (see Fig. 7). Then, at step S201, the drive circuit 303 causes the heating resistor 61 (see Figs. 1 and 2) to heat at 100°C and the heat dissipation constant calculation module 301 (see Fig. 7) calculates a heat dissipation constant value at the heating temperature 100°C. Then, the heat dissipation constant calculation module 301 stores, in the heat dissipation constant storage device 401, the heat dissipation constant value of the measurement subject mixed gas at the heating temperature 100°C.

(b) At step S202, it is judged whether the switching of the heating temperature of the heating resistor 61 (see Figs. 1 and 2) has completed. Since switching to the heating temperature 150°C or the heating temperature 200°C has not been made yet, the process returns to step S201, where the drive circuit 303 causes the heating resistor 61 (see Figs. 1 and 2) to heat at 150°C. The heat dissipation constant calculation module 301 (see Fig. 7) calculates a heat dissipation constant value of the measurement subject mixed gas at the heating temperature 150°C and stores it in the heat dissipation constant storage device 401.

(c) Again at step S202, it is judged whether the switching of the heating temperature of the heating resistor 61 (see Figs. 1 and 2) has completed. Since switching to the heating temperature 200°C has not been made yet, the process returns to step S201, where the drive circuit 303 causes the heating resistor 61 (see Figs. 1 and 2) to heat at 200°C. The heat dissipation constant calculation module 301 (see Fig. 7) calculates a heat dissipation constant value of the measurement subject mixed gas at the heating temperature 200°C and stores it in the heat dissipation constant storage device 401.

(d) At step S203, the calorific value calculation module 305 reads out a calorific value calculation formula having the heat dissipation constant at the heating temperatures 100°C, 150°C, and 200°C as independent variables from the formula storage device 402. The calorific value calculation module 305 reads out the heat dissipation constant values of the measurement subject mixed gas at the respective heating temperatures 100°C, 150°C, and 200°C from the heat dissipation constant storage device 401.

(e) At step S204, the calorific value calculation module 305 calculates a calorific value of the measurement subject mixed gas by substituting the heat dissipation constant values of the measurement subject mixed gas at the respective heating temperatures 100°C, 150°C, and 200°C into the independent variables of the calorific value calculation formula. Then, the calorific value calculation module 305 stores the calculated calorific value in the calorific value storage device 403. The calorific value calculating method according to the second embodiment is thus finished.

The above-described calorific value calculating system and method according to the second embodiment make it possible to measure a calorific value of a mixed gas merely by measuring heat dissipation constant values without using an expensive gas chromatography instrument or sound speed sensor.

The proportions of hydrocarbon components of the natural gas depend on the gas field where it is produced. And the natural gas contains nitrogen ($N_2$), carbon dioxide ($CO_2$), etc. in addition to hydrocarbons. Therefore, the volume proportions of gas components of the natural gas depend on the gas field where it is produced. Even if the kinds of gas components of a natural gas are known, in many cases its calorific value is unknown. Even natural gases originating from the same gas field do not always have a constant calorific value and the calorific value may vary depending on the period of production.

As a result, conventionally, in collection of use fees of natural gas, a user is charged according to a use volume of natural gas rather than a use calorific value. However the charging according to the use volume is not fair because the calorific value of the natural gas depends on the gas field where it is produced. In contrast, the calorific value calculating system and method according to the second embodiment make it possible to measure, in a simple manner, a calorific value of a mixed gas such as a natural gas whose calorific value is unknown because its volume proportions of gas components are unknown though its kinds of gas components are known. The calorific value calculating system and method according to the second embodiment thus enable fair collection of use fees.

Furthermore, since it becomes possible to know a correct calorific value easily, an air amount that is necessary for burning a mixed gas can be set properly and the discharge amount of uselessly discharged carbon dioxide ($CO_2$) can be reduced.

(Example)

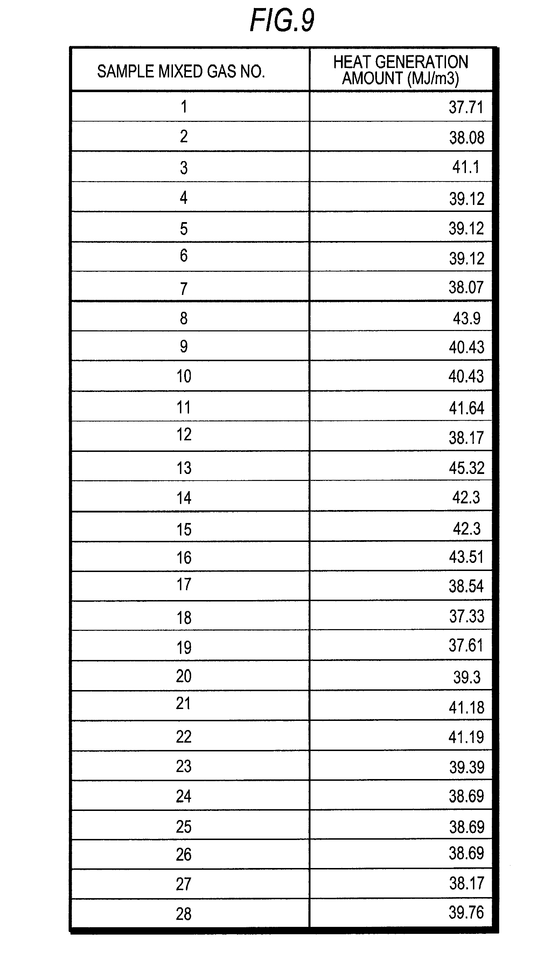

First, 28 kinds of sample mixed gases were prepared whose calorific values are known as shown in Fig. 9. Each of the 28 kinds of sample mixed gases contained, as gas components, all or part of methane ($CH_4$), ethane ($C_2H_6$), propane ($C_3H_8$), butane ($C_4H_{10}$), nitrogen ($N_2$), and carbon dioxide ($CO_2$). For example, the No. 7 sample mixed gas contained methane at 90 vol%, ethane at 3 vol%, propane at 1 vol%, butane at 1 vol%, nitrogen at 4 vol%, and carbon dioxide at 1 vol%. The No. 8 sample mixed gas contained methane at 85 vol%, ethane at 10 vol%, propane at 3 vol%, and butane at 2 vol% and did not contain nitrogen or carbon dioxide. The No. 9 sample mixed gas contained methane at 85 vol%, ethane at 8 vol%, propane at 2 vol%, butane at 1 vol%, nitrogen at 2 vol%, and carbon dioxide at 2 vol%. Then, heat dissipation constant values of each of the 28 sample mixed gases were measured at 100°C, 150°C, and 200°C. Although the No. 7 sample mixed gas, for example, contains six kinds of gas components, measuring heat dissipation constant values at the three temperatures raises no problems because, as described above, each of ethane ($C_2H_6$) and butane ($C_4H_{10}$) can be regarded as a mixture of methane ($CH_4$) and propane ($C_3H_8$). Then, a linear equation, a quadratic equation, and a cubic equation for calculating a calorific value which have the heat dissipation constant as independent variables and the calorific value as an dependent variable were generated by support vector regression on the basis of the calorific values and measured heat dissipation constant values of the 28 kinds of sample mixed gases.

In generating a linear equation for calculating a calorific value, calibration points can be determined as appropriate (a rough measure of the number of calibration points is three to five). A generated linear equation is given by the following Equation (23). Calorific values of the 28 sample mixed gases were calculated according to Equation (23) and compared with the true calorific values to find that the maximum error was 2.1%.

$$Q = 39.91 - 20.59 \times M_1(100°C) - 0.89 \times M_1(150°C)$$

$$+ 19.73 \quad \times M_1(200°C) \quad \cdots (23)$$

In generating a quadratic equation for calculating a calorific value, calibration points can be determined as appropriate (a rough measure of the number of calibration points is eight and nine). Calorific values of the 28 sample mixed gases were calculated according to the generated quadratic equation and compared with the true calorific values to find that the maximum error was 1.2% to 1.4%.

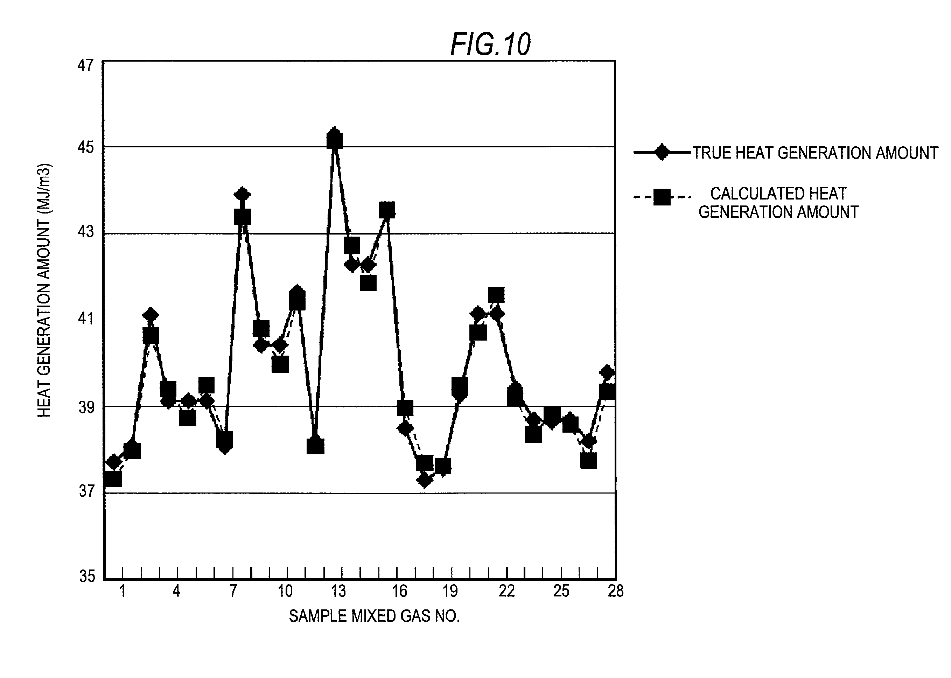

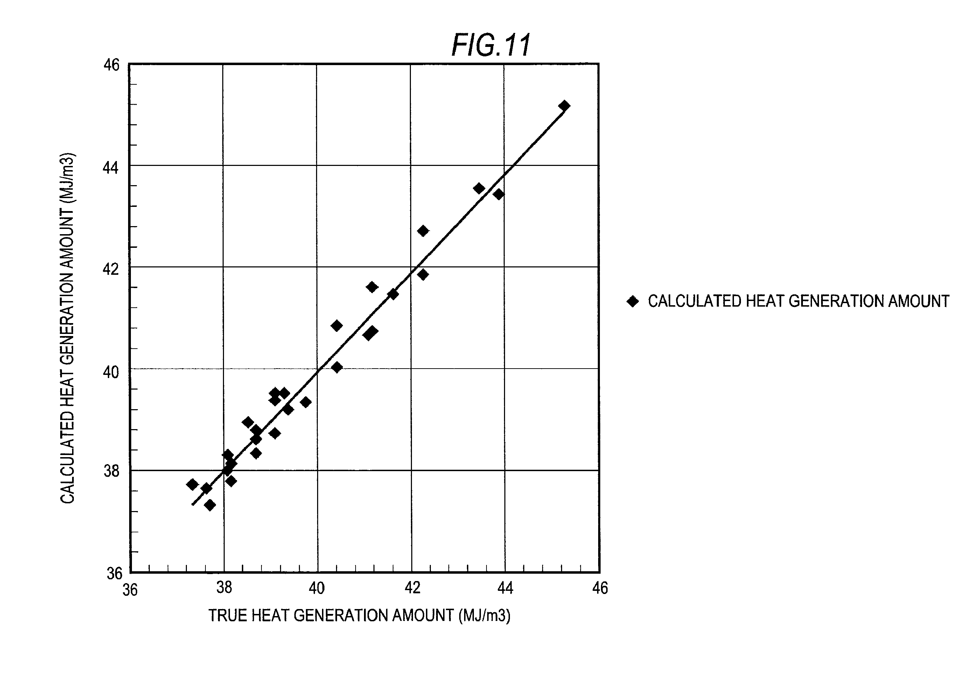

In generating a cubic equation for calculating a calorific value, calibration points can be determined as appropriate (a rough measure of the number of calibration points is 10 to 14). Calorific values of the 28 sample mixed gases were calculated according to the generated cubic equation and compared with the true calorific values to find that the maximum error was less than 1.2%. As seen from Figs. 10 and 11, the calorific values calculated according to the cubic equation which was generated by taking 10 calibration points well approximated the true calorific values.

(Other embodiments)

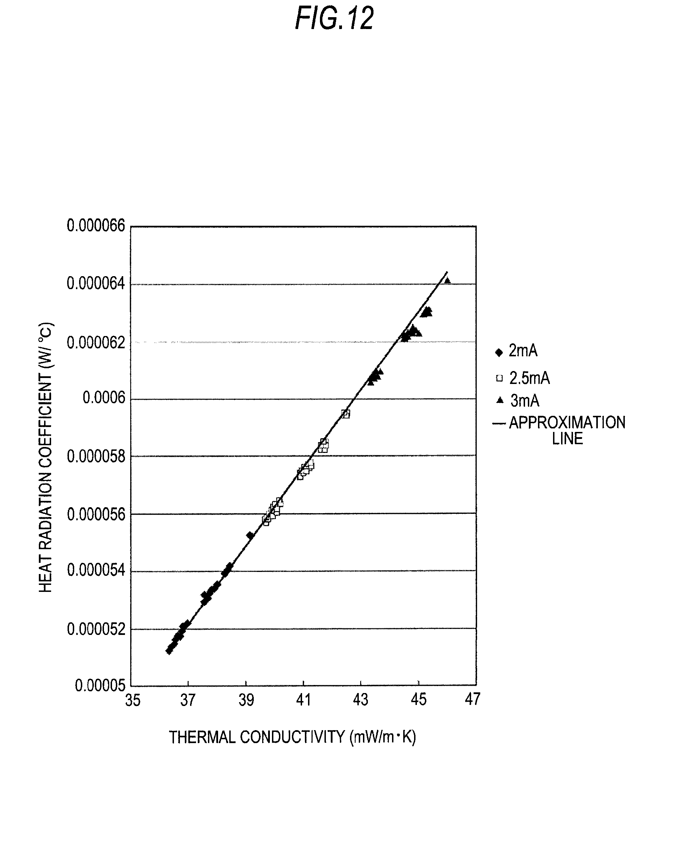

Although the invention has been described above by means of the embodiments, the above description and the drawings which are part of this disclosure should not be construed as restricting the invention. Various replacement embodiments, other embodiments, and operation techniques will become apparent to a person skilled in the art from this disclosure. For example, Fig. 12 shows a relationship between the heat dissipation constant and the thermal conductivity of a mixed gas when currents of 2 mA, 2.5 mA, and 3 mA are caused to flow through the heating resistor. As seen from Fig. 12, in general, the heat dissipation constant and the thermal conductivity of a mixed gas have a linear relationship. Therefore, whereas the first and second embodiments employ heat dissipation constant values of each mixed gas at plural heating temperatures of the heating resistor, generation of a calorific value calculation formula and calculation of a calorific value may be done using thermal conductivity values at plural measurement temperatures of each mixed gas. As exemplified by this, it should be understood that the invention encompasses various embodiments etc. that are not described in this specification. As such, the invention is restricted only by the invention-defining items in the claims which are suitable for the disclosure.

[Industrial applicability]

The calorific value calculation formula generating system, the calorific value calculation formula generating method, the calorific value calculating system, and the calorific value calculating method of the invention can be applied to the energy industry etc.

**Claims**

1. A calorific value calculation formula generating system comprising:

   a measuring mechanism for measuring, at plural temperatures, heat dissipation constant values or thermal conductivity values of each of plural mixed gases each containing plural kinds of gas components; and
   a formula generation module for generating a calorific value calculation formula having heat dissipation constant or thermal conductivities at the plural temperatures as independent variables and a calorific value as a dependent variable on the basis of known calorific value values of the plural mixed gases and the heat dissipation constant values or the thermal conductivity values measured at the plural temperatures.

2. The calorific value calculation formula generating system according to claim 1, wherein the number of the plural temperatures is at least the number of the plural kinds of gas components minus 1.

3. The calorific value calculation formula generating system according to claim 1 or 2, wherein the formula generation module generates a calorific value calculation formula using support vector regression.

4. The calorific value calculation formula generating system according to any one of claims 1 to 3, wherein the measuring mechanism comprises a heater for heating each of the plural mixed gases.

**5.** The calorific value calculation formula generating system according to claim 4, wherein the measuring mechanism further comprises a heat dissipation constant calculation module for calculating heat dissipation constant values of each of the plural mixed gases by dividing drive power values of the heater by differences between temperatures of the heater and temperatures of each of the plural mixed gases, respectively.

**6.** The calorific value calculation formula generating system according to any one of claims 1 to 5, wherein each of the plural mixed gases is a natural gas.

**7.** The calorific value calculation formula generating system according to any one of claims 1 to 6, wherein each of the plural mixed gases contains methane, propane, nitrogen, and carbon dioxide as the plural kinds of gas components.

**8.** A calorific value calculation formula generating method comprising the steps of:

preparing plural mixed gases each containing plural kinds of gas components;
measuring heat dissipation constant values or thermal conductivity values of each of the plural mixed gases at plural temperatures; and
generating a calorific value calculation formula having heat dissipation constant or thermal conductivities at the plural temperatures as independent variables and a calorific value as a dependent variable on the basis of known calorific value values of the plural mixed gases and the heat dissipation constant values or the thermal conductivity values measured at the plural temperatures.

**9.** The calorific value calculation formula generating method according to claim 8, wherein the number of the plural temperatures is at least the number of the plural kinds of gas components minus 1.

**10.** The calorific value calculation formula generating method according to claim 8 or 9, wherein the step of generating a calorific value calculation formula uses support vector regression.

**11.** The calorific value calculation formula generating method according to any one of claims 8 to 10, wherein the step of measuring heat dissipation constant values of each of plural mixed gases comprises heating each of the plural mixed gases with a heater, and dividing drive power values of the heater by differences between temperatures of the heater and temperatures of each of the plural mixed gases, respectively.

**12.** The calorific value calculation formula generating method according to any one of claims 8 to 11, wherein each of the plural mixed gases is a natural gas.

**13.** The calorific value calculation formula generating method according to any one of claims 8 to 12, wherein each of the plural mixed gases contains methane, propane, nitrogen, and carbon dioxide as the plural kinds of gas components.

**14.** A calorific value calculating system comprising:

a measuring mechanism for measuring, at plural temperatures, heat dissipation constant values or thermal conductivity values of a measurement subject mixed gas whose calorific value is unknown;
a formula storage device for storing a calorific value calculation formula having heat dissipation constant or thermal conductivities at the plural temperatures as independent variables and a calorific value as a dependent variable; and
a calorific value calculation module for calculating a calorific value of the measurement subject mixed gas by substituting the heat dissipation constant values or the thermal conductivity values of the measurement subject mixed gas measured at the plural temperatures into the heat dissipation constant or the thermal conductivities at the plural temperatures as the independent variables of the calorific value calculation formula.

**15.** The calorific value calculating system according to claim 14, wherein the number of the plural temperatures is at least the number of the plural kinds of gas components contained in the measurement subject mixed gas minus 1.

**16.** The calorific value calculating system according to claim 14 or 15, wherein the calorific value calculation formula having the heat dissipation constant or the thermal conductivities at the plural temperatures as the independent variables and the calorific value as the dependent variable was generated on the basis of calorific value values of plural sample mixed gases each containing plural kinds of gas components and heat dissipation constant values or thermal conductivity values of each of the plural sample mixed gases measured at the plural temperatures.

17. The calorific value calculating system according to claim 16, wherein support vector regression was used for generating the calorific value calculation formula.

18. The calorific value calculating system according to any one of claims 14 to 17, wherein the measuring mechanism comprises a heater for heating the measurement subject mixed gas.

19. The calorific value calculating system according to claim 18, wherein the measuring mechanism further comprises a heat dissipation constant calculation module for calculating heat dissipation constant values of the measurement subject mixed gas by dividing drive power values of the heater by differences between temperatures of the heater and temperatures of the measurement subject mixed gas, respectively.

20. The calorific value calculating system according to claim 16 or 17, wherein the each of the plural sample mixed gases is a natural gas.

21. The calorific value calculating system according to any one of claims 16, 17, and 20, wherein each of the plural sample mixed gases contains methane, propane, nitrogen, and carbon dioxide as the plural kinds of gas components.

22. The calorific value calculating system according to any one of claims 14 to 21, wherein the measurement subject mixed gas is a natural gas.

23. The calorific value calculating system according to any one of claims 14 to 22, wherein the measurement subject mixed gas contains methane, propane, nitrogen, and carbon dioxide.

24. The calorific value calculating system according to claim 23, wherein the measurement subject mixed gas further contains an alkane.

25. A calorific value calculating method comprising the steps of:

measuring, at plural temperatures, heat dissipation constant values or thermal conductivity values of a measurement subject mixed gas whose calorific value is unknown;
preparing a calorific value calculation formula having heat dissipation constant or thermal conductivities at the plural temperatures as independent variables and a calorific value as a dependent variable; and
calculating a calorific value of the measurement subject mixed gas by substituting the heat dissipation constant values or the thermal conductivity values of the measurement subject mixed gas measured at the plural temperatures into the heat dissipation constant or the thermal conductivities at the plural temperatures as the independent variables of the calorific value calculation formula.

26. The calorific value calculating method according to claim 25, wherein the number of the plural temperatures is at least the number of the plural kinds of gas components contained in the measurement subject mixed gas minus 1.

27. The calorific value calculating method according to claim 25 or 26, wherein the calorific value calculation formula having the heat dissipation constant or the thermal conductivities at the plural temperatures as the independent variables and the calorific value as the dependent variable was generated on the basis of calorific value values of plural sample mixed gases each containing plural kinds of gas components and heat dissipation constant values or thermal conductivity values of each of the plural sample mixed gases measured at the plural temperatures.

28. The calorific value calculating method according to claim 27, wherein support vector regression was used for generating the calorific value calculation formula.

29. The calorific value calculating method according to any one of claims 25 to 28, wherein the step of measuring radiation coefficient values of the measurement subject mixed gas comprises heating the measurement subject mixed gas with a heater, and dividing drive power values of the heater by differences between temperatures of the heater and temperatures of the measurement subject mixed gas, respectively.

30. The calorific value calculating method according to claim 27 or 28, wherein the each of the plural sample mixed gases is a natural gas.

**31.** The calorific value calculating method according to any one of claims 27, 28, and 30, wherein each of the plural sample mixed gases contains methane, propane, nitrogen, and carbon dioxide as the plural kinds of gas components.

**32.** The calorific value calculating method according to any one of claims 25 to 31, wherein the measurement subject mixed gas is a natural gas.

**33.** The calorific value calculating method according to any one of claims 25 to 32, wherein the measurement subject mixed gas contains methane, propane, nitrogen, and carbon dioxide.

**34.** The calorific value calculating method according to claim 33, wherein the measurement subject mixed gas further contains an alkane.

# FIG.1

EP 2 345 891 A1

FIG.2

## FIG.3

EP 2 345 891 A1

# FIG.4

20

HEAT RADIATION COEFFICIENT STORAGE DEVICE — 401

FORMULA STORAGE DEVICE — 402

300

CPU

301

HEAT RADIATION COEFFICIENT CALCULATION MODULE

302

FORMULA GENERATION MODULE

INPUT DEVICE — 312

OUTPUT DEVICE — 313

DRIVE CIRCUIT

303

301
8 } 10

101

8

102    103

# FIG.5

## FIG.6

```
                    ┌─────────┐
                    │  START  │
                    └────┬────┘
                         │
    ┌──────────────────→ │
    │                    ▼
    │          ┌─────────────────────────┐
    │          │  PREPARE SAMPLE MIXED GAS│────── S100
    │          └────────────┬────────────┘
    │                       │
    │    ┌────────────────→ │
    │    │                  ▼
    │    │        ┌─────────────────────┐
    │    │        │ MEASURE HEAT RADIATION│──── S101
    │    │        │  COEFFICIENT VALUE   │
    │    │        └──────────┬──────────┘
    │    │                   │
    │    │                   ▼                  S102
    │    │   NO      ◇ HAS TEMPERATURE ◇
    │    └──────────  SWITCHING COMPLETED?
    │                        │
    │                       YES
    │                        │
    │                        ▼                  S103
    │          NO     ◇ HAS SWITCHING OF ◇
    └─────────────── SAMPLE MIXED GAS COMPLETED?
                             │
                            YES
                             │
                             ▼
                   ┌──────────────────┐
                   │   COLLECT DATA   │────── S104
                   └────────┬─────────┘
                            │
                            ▼
                   ┌──────────────────┐
                   │ PERFORM MULTIPLE │────── S105
                   │ REGRESSION ANALYSIS│
                   └────────┬─────────┘
                            │
                            ▼
                   ┌──────────────────┐
                   │   REGISTRATION   │────── S106
                   └────────┬─────────┘
                            │
                            ▼
                       ┌─────────┐
                       │   END   │
                       └─────────┘
```

# FIG.7

# FIG.8

START

PREPARE MEASUREMENT SUBJECT MIXED GAS —— S200

MEASURE HEAT RADIATION COEFFICIENT. —— S201

HAS TEMPERATURE SWITCHING COMPLETED? —— S202

NO

YES

READ OUT FORMULA —— S203

CALCULATE HEAT GENERATION AMOUNT —— S204

END

## FIG.9

| SAMPLE MIXED GAS NO. | HEAT GENERATION AMOUNT (MJ/m3) |
|---|---|
| 1 | 37.71 |
| 2 | 38.08 |
| 3 | 41.1 |
| 4 | 39.12 |
| 5 | 39.12 |
| 6 | 39.12 |
| 7 | 38.07 |
| 8 | 43.9 |
| 9 | 40.43 |
| 10 | 40.43 |
| 11 | 41.64 |
| 12 | 38.17 |
| 13 | 45.32 |
| 14 | 42.3 |
| 15 | 42.3 |
| 16 | 43.51 |
| 17 | 38.54 |
| 18 | 37.33 |
| 19 | 37.61 |
| 20 | 39.3 |
| 21 | 41.18 |
| 22 | 41.19 |
| 23 | 39.39 |
| 24 | 38.69 |
| 25 | 38.69 |
| 26 | 38.69 |
| 27 | 38.17 |
| 28 | 39.76 |

FIG.10

EP 2 345 891 A1

FIG.11

EP 2 345 891 A1

## FIG.12

## INTERNATIONAL SEARCH REPORT

International application No.

PCT/JP2008/067811

| A. CLASSIFICATION OF SUBJECT MATTER |
| --- |
| G01N25/18(2006.01)i |

According to International Patent Classification (IPC) or to both national classification and IPC

| B. FIELDS SEARCHED |
| --- |
| Minimum documentation searched (classification system followed by classification symbols)<br>G01N25/18 |

| Documentation searched other than minimum documentation to the extent that such documents are included in the fields searched |
| --- |
| Jitsuyo Shinan Koho        1922-1996   Jitsuyo Shinan Toroku Koho   1996-2008<br>Kokai Jitsuyo Shinan Koho   1971-2008   Toroku Jitsuyo Shinan Koho   1994-2008 |

| Electronic data base consulted during the international search (name of data base and, where practicable, search terms used) |
| --- |
| JSTPlus(JDreamII) |

C. DOCUMENTS CONSIDERED TO BE RELEVANT

| Category* | Citation of document, with indication, where appropriate, of the relevant passages | Relevant to claim No. |
| --- | --- | --- |
| A | JP 8-75688 A  (Yamatake-Honeywell Co., Ltd.),<br>22 March, 1996 (22.03.96),<br>Full text; all drawings<br>(Family: none) | 1-34 |
| A | JP 2007-248220 A  (Yamatake Corp.),<br>27 September, 2007 (27.09.07),<br>Full text; all drawings<br>(Family: none) | 1-34 |
| A | JP 2004-514138 A  (LATTICE INTELLECTUAL<br>PROPERTY LTD.),<br>13 May, 2004 (13.05.04),<br>Full text; all drawings<br>& US 2004/0261497 A1     & GB 2372819 A<br>& EP 1337844 A           & WO 2002/040992 A1 | 1-34 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>    16 December, 2008 (16.12.08) | Date of mailing of the international search report<br>    22 December, 2008 (22.12.08) |
| --- | --- |
| Name and mailing address of the ISA/<br>    Japanese Patent Office | Authorized officer |
| Facsimile No. | Telephone No. |

Form PCT/ISA/210 (second sheet) (April 2007)

**INTERNATIONAL SEARCH REPORT**

| International application No. |
|---|
| PCT/JP2008/067811 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 8-50109 A  (Yamatake-Honeywell Co., Ltd.), 20 February, 1996 (20.02.96), Full text; all drawings (Family: none) | 1-34 |
| A | Hiroyuki MUTO, Yasuhiro KAJIO, Minoru SETO, "Netsudendoritsushiki Bunsekikei ni yoru Toshi Gas Hatsunetsuryo Sokutei", Savemation Rev, 01 February, 1995 (01.02.95), Vol.13, No.1, pages 35 to 39 | 1-34 |
| A | Yasuharu OISHI, Shigeru AOSHIMA, Yasue HAYASHI, Nobuyoshi SHINGYOJI, "Tennen Gas no Netsuryo Ryuryo Keisoku no Kanosei ni Tsuite", Proceedings of Sensing Forum, 2005, Vol.22nd, pages 371 to 375 | 1-34 |

Form PCT/ISA/210 (continuation of second sheet) (April 2007)

# EP 2 345 891 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004514138 T
- JP 5141999 A